# EUROPEAN PATENT SPECIFICATION

(11) **EP 1 592 469 B1**
(45) Date of publication and mention of the grant of the patent: **17.12.2008**
(21) Application number: 04709986.6
(22) Date of filing: 11.02.2004
(51) Int. Cl.: A61M 5/158, A61M 25/02, A61M 25/00

(54) **A COVER FOR AN INFUSION SITE**
ABDECKUNG EINER INFUSIONSSTELLE
COUVERCLE POUR SITE DE TRANSFUSION

(30) Priority: 12.02.2003 DK 200300206; 12.02.2003 US 366453
(43) Date of publication of application: 09.11.2005
(73) Proprietor: Unomedical A/S, 3460 Birkerød (DK)
(72) Inventor: JENSEN, Soren, 3540 Lynge (DK)
(74) Representative: Münzer, Marc Eric
(86) International application number: PCT/DK2004/000098
(87) International publication number: WO 2004/071556

(56) References cited:
- WO-A-87/06474
- US-A- 5 112 313
- US-A- 5 116 324
- US-A- 5 522 803
- US-A1- 2002 068 904

## Description

The invention relates to a cover for being located on an infusion device, said cover comprising an upper plate element with an upper face and a lower face oriented towards the upper face of the infusion device.

In connection with an infusion device being located on the skin, it is known to shield it; such shielding, however, involving a complete covering of the infusion device and in such a manner that it becomes inaccessible to manipulation from the outside.

One example of this is known for instance from WO 87/06474. This discloses a device that can be strapped to the body over the location of insertion of an intravenous needle to shield the puncture area against contact and for anchoring the tube so that the needle will not be pulled out inadvertently.

However the construction necessitates use of an additional frame for securing the infusion device and also for securing the dome that shields the entire infusion device and is also manufactured to be very voluminous. There is thus a risk of it being torn off during use and, likewise, it prevents access to the infusion device and, furthermore, there is a risk of the adhesive effect ceasing and, likewise, it cannot be expected that adjustments of the shielding are possible as the adhesive effect will cease.

It is thus the object of the present invention as claimed to provide a cover that remedies the problems that may occur in convection with the prior art covers and whereby complete or partial shielding of the upper free face of the infusion set is enabled, but wherein the lower face is unshielded and the side faces are also partially unshielded, and wherein a shielding of the plate takes place directly on the infusion device. Hereby it is obtained that the risk of bacteria and soil collected thereon penetrating into the construction is reduced considerably, and likewise the cover enables a smoother surface with an ensuing minimisation of the risk of the infusion device being torn off.

This object is obtained by a cover like the one described in the preamble to claim 1 and wherein the lower face of the plate element also comprises attachment means, said means co-operating with elements on the infusion device.

Thus, the cover functions in that, by means of its attachment means that are provided on the lower free face, it fits into the upper face of the infusion device, and by a press fitting it is ensured that the cover is securely attached onto the infusion device due to the clamping effect obtained between the individual attachment means which can be reinforced by the infusion device, the cover preferably comprising, as described in the attached claims, a side flange, wherein this side flange cooperates with the attachment means to bring about a secure attachment of the cover on the infusion device.

Preferably the upper face of the infusion device is configured with recesses, on the one hand longitudinally extending recesses and on the other hand rather rounded cavities. Consequently the attachment means of the cover match these cavities and bring about the attachment. The upper face of the cover is generally smooth and the entire lower face of the cover is essentially congruent with the upper face of the infusion device, whereby the adaptation is optimised. The cover may be removed by seizing side flanges, if any, disposed on the cover thereby exposing the entire infusion device. The cover can be configured such that the upper plate does not cover the entire infusion device, but merely comprises a tongue portion that extends across a portion of the upper face of the infusion device and an essentially partially circular part that covers the remainder.

The cover according to the invention is thus a separate part comprising integrated means for attaching the cover to the infusion device. Thus it is further accomplished that the cover, when removed allows easy access to operate the infusion device.

By providing a cover according to the invention and as further featured in claim 2, it is obtained that, the connection between the interconnectable parts of the infusion device can be shielded from contamination.

By the cover only covering a part of the connection, more specifically the liquid connection parts between the cannula housing and the connector hub, the liquid connection can be shielded from contamination, while still allowing access to release means for disconnecting the cannula housing from the connector hub as obtained by the features revealed in claim 3.

By providing a cover according to the invention and as further featured in claim 4, it is obtained that, in case the infusion device that consists of two parts is separated, it is possible - due to the presence of the flange - for the cover to remain in place on the part designated the cannula housing; the attachment means and the flange bringing about a squeezing effect that secures the cover in position.

By provision of a cover in accordance with the invention and as further featured in claim 5, an optimal complete or partial shielding of the infusion device is obtained.

Provision of a cover according to the invention and as further featured in claim 6 makes it possible to seize the exposed parts of the infusion device and thus to carry out a separation thereof.

Provision of a cover in accordance with the invention and as further featured in claim 7 enables close contact between the cover and the infusion device, and hereby a snap-on attachment is obtained.

Provision of a cover in accordance with the invention and as further featured in claims 8, 9 and 10 brings about a convenient shape of the attachment means.

Provision of a cover in accordance with the invention and as further featured in claim 11 enables separation of the hub and cannula part while simultaneously the cover remains in place on the cannula part.

The invention as claimed will now be explained in further detail with reference to the drawing, wherein
Figures 1-4 show an infusion set as described in US 5522803 and for which a cover in accordance with the invention can be used;
Figures 5A-B show a first exemplary embodiment of a cover according to the invention, seen in a perspective view from above at two different angles;
Figure 6A-B show the cover shown in Figure 5, in a perspective view seen from below, and essentially from the side;
Figure 7 shows an alternative embodiment according to the invention, seen in perspective view from the bottom;
Figure 8 shows a third exemplary embodiment in accordance with the invention, seen in perspective view and from the bottom;
Figure 9 shows a fourth exemplary embodiment, seen in perspective view and from the bottom;
Figure 10 shows the cover shown in Figure 5 located on an infusion set in accordance with Figure 1.

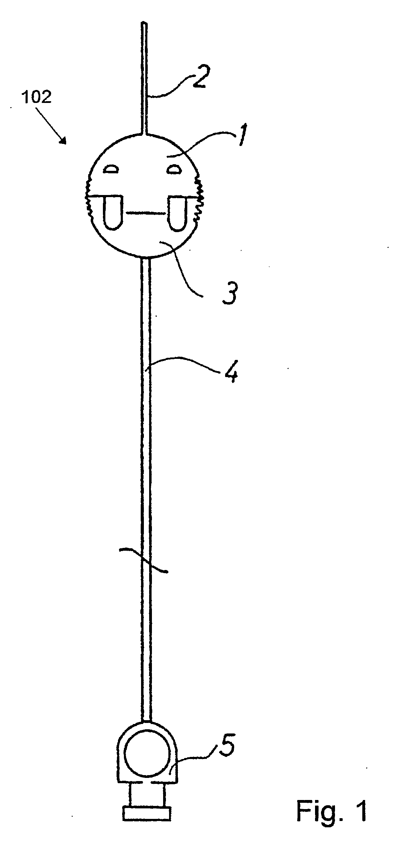

The infusion set 102 of Figure 1 comprises a cannula housing 1 with a soft cannula 2 secured therein and manufactured in a conventional manner of a suitable plastics material. The infusion set 102 further comprises a connecting hub 3 connected in a conventional manner through a hose 4 with a luer coupling member 5 to be used for the coupling thereof to an insulin pump (not shown).

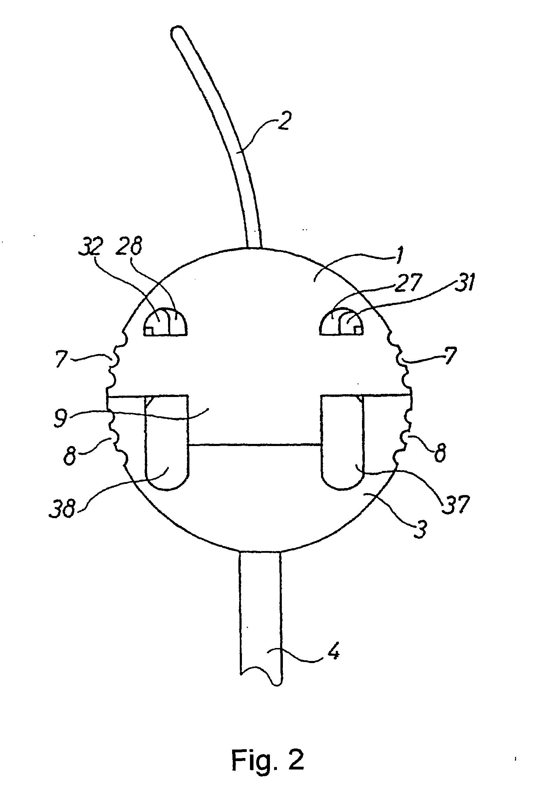

Figure 2 shows the cannula housing 1 and the connecting hub 3 on a larger scale, constituting in combination the infusion device 102. It appears clearly that the combination constitutes a relatively flat shape and of a substantially uniform thickness having a substantially plane rear side adapted for abutment to the skin of a patient. When seen from the top, the combination constitutes a substantially circular shape, said shape being interrupted only by recesses 7 and 8, respectively, for facilitating a finger-handling. As illustrated in Figure 2, the cannula housing 1 and the connecting hub 3 are divided substantially along a diametrical central plane, a central projection 9 being provided on said cannula housing 1 and projecting into a mating recess in the connecting hub 3. In Figure 2, the cannula 2 is slightly curved in order to illustrate that it is constituted by a soft, bendable tubing.

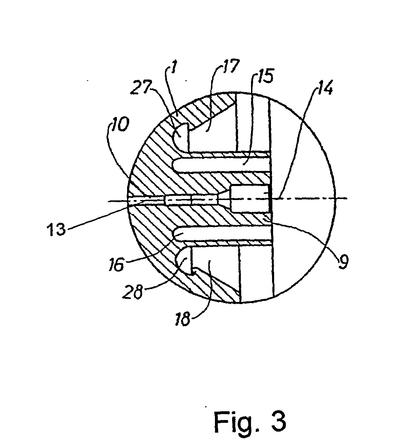

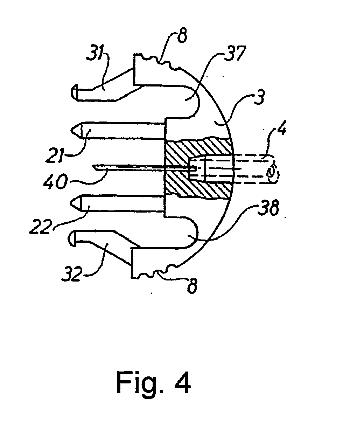

As illustrated in Figure 3, the cannula housing 1 comprises two guide openings 15 and 16 and two locking openings 17 and 18 in addition to the rectilinear through bore 10. These openings are symmetrically shaped about a plane including the central axis 14 of the through passageway 13 and extending perpendicular to the generally planar rear side of the injection device adapted for abutment against the skin of a patient. The guide openings 15 and 16 are elongate openings, adapted to receive mating guide pins 21 and 22 on the connecting hub 3, cf. Figure 4.

The locking openings 17 and 18 adjacent the guide openings 15 and 16 and the central through bore 10 follow a rectilinear course parallel to the central axis 14 and consequently also parallel to the guide openings 15 and 16. The locking openings 17 and 18 are at the bottom connected to the surface of the cannula housing through shafts or channels 27 and 28, respectively, extending perpendicular from said locking openings 17 and 18.

The locking openings 17 and 18 are adapted to receive mating symmetrically shaped locking pins 31 and 32 on the connecting hub 3. As can be appreciated by comparing figures 3 and 4 with figure 2, these locking pins 31 and 32 are adapted for engagement with the locking openings 17 and 18.

As illustrated in Figures 2 and 4, curve-shaped recesses 37 and 38 are shaped on each side of the guide pins 21 and 22. These recesses ensure that the locking pins 31 and 32 can be moved easily by means of a finger.

Furthermore, figure 4 shows that centrally the connecting hub 3 comprises a needle 40. When inserted through a membrane in the cannula housing 1, this needle provides a connection between the hose 4 of the infusion set and the through passageway 13 of the cannula housing 1.

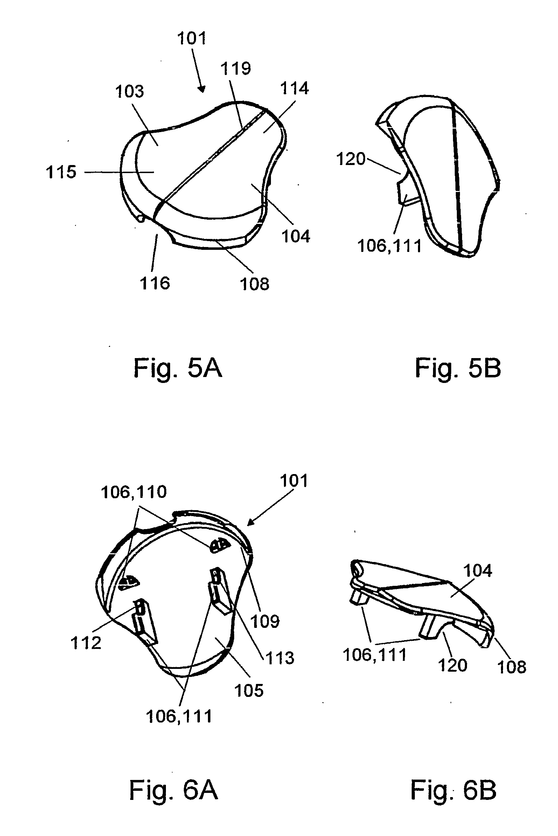

Reference now being made to Figures 5-9, the cover 101 will be described in further detail. Figure 5 shows a first exemplary embodiment of a cover 101 seen from the top and comprising an upper plate portion 103 and has an upper face 104 which is essentially plane or optionally slightly curved and generally smooth. In this case, the upper plate portion 103 is configured as a partially circular element 115 that extends into a tongue part 114. At the delimiting edge of the plate element corresponding to the partially circular area, it extends into a flange 108, wherein flange and plate element form an essentially 90° wide angle - a first angle - to each other. Furthermore, the flange 108 has a partially circular recess 116 essentially corresponding to the centre axis 119 of the cover and arranged symmetrically thereabout, said recess 116 ensuring that cannulas can freely and unimpededly extend from whatever infusion device 102 shielded by the cover 101. Thus the cover is configured symmetrically about its longitudinally extending centre axis 119. Typically the upper plate portion 103 will have a thickness within the range 0.5-2 mm. Typically the flange 108 will assume approximately the same thickness as the upper plate portion 103. However, sections of the flange and/or the upper plate portion 103 may optionally be thicker, e.g. in the transition area between plate and flange.

Figure 6A in a principle outline shows the cover shown in Figure 5, seen from below, from where the lower face 105 of the upper plate portion 103 will appear which is essentially plane, optionally slightly curved; such, however, that this face 105 is congruent with the upper surface of the infusion device 102 on top of which the cover 101 is to be located: This face 105 comprises attachment means 106 that comprise in this case two types, viz two plate elements 111 and two partially cylindrical elements 110. The two plate elements 111 are each configured as rectangular plate elements that are cast integrally with the cover and are located perpendicular to the lower face 105. The rectangular faces of the plate elements are in parallel with the centre axis 119 of the cover and are located with a second mutual spacing, corresponding to 9-10 mm. The plate elements are located in such a manner that, when the cover is located on an infusion device, they will fit into recesses on the infusion device, eg the recesses indicated in Figure 2 by 37 and 38. Hereby a firm attachment of the cover to the infusion device is ensured, since - on the one hand - there is a span between the two rectangular plate elements 111 and - on the other - a span between flange 108 and plate elements 111.

Conveniently each of the plate elements 111 can be provided with a recess: a partially circular recess 120 facing towards the flange 108 and corresponding to the upper free edge, whereby a nail-shaped cavity 120 is provided and being shown in figure 6B with a concave face 113 that extends into the side faces at a concave delimiting edge 112. It is the object of this nail-shaped recess 120 to enable the cover to remain in place when the two parts of the infusion device 102, viz the cannula housing 1 and the connection hub 3, are separated and wherein the cover 101 remains in position on the cannula housing. The connector hub 3 is removed from the cannula housing 1 with the cover 101 attached thereto, by pressing the handles 8 disposed on each side of connector hub 3. Thereby the locking pins 31, 32 are - due to their inherent resiliency - temporarily relocated towards the centre of the connector hub 3, disengaging taps disposed at the end of the locking pins 31, 32 from recesses 27, 28 disposed in the cannula housing 1 to release the locking engagement of the connector hub 3 to the cannula housing 1. The nail-shaped recesses 120 thus provides space to allow said sideways movement of locking pins 31, 32. The connector hub can thus be removed, while the cannula housing remains on the patient, the soft cannula 2 inserted, and the cover 102 protecting the fluid connection part of the cannula housing 1 and the cannula housing 1 as such.

Furthermore, in this exemplary embodiment the attachment means 106 also comprise the two partially cylindrical elements 110 extending perpendicularly to the lower face 105 and located at a first mutual distance in such a manner that they fit into the openings or channels 27, 28 on the infusion device 102 that are shown in Figure 2. The height of these partially cylindrical attachment means 110 is typically in an order of magnitude of 1 mm. The height above the lower surface 105 of the rectangular plate elements 111 is typically 3.5 mm, whereas the length relative to a direction generally parallel to the central axis 119 of the latter and measured at the base thereof is comprised within the range 5-6 mm.

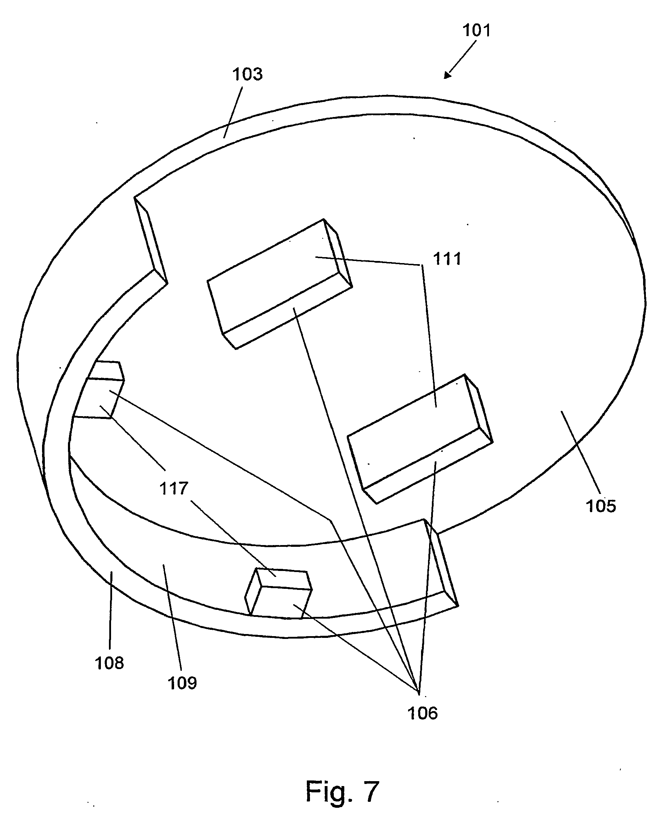

Figure 7 shows a further exemplary embodiment of a cover 101 and wherein the difference over those described in Figures 5 and 6 resides in the shape of the attachment means and also the geometrical shape of the upper plate part 103. In this case the upper plate element 103 is shaped to be circular and, likewise, the flange is not provided with a recess 116. The rectangular plate elements are provided on the same location relative to the infusion device as in Figures 5-6, but they do not comprise the concave delimiting edge 112, but constitute a true rectangular plate. Thus in this embodiment the rectangular face elements 111 will prevent the locking pins 31, 32 to be relocated if the handles 8 on the connector hub 3 are pressed, by blocking said movement. This can be appreciated by comparing figures 7 and 4. In this embodiment the connector hub 3 is thus prevented from being disconnected from the cannula housing. Further attachment means are in this case located on the flange, the inner face 109 of the flange being provided with two blocks or taps 117, whose upper and lower delimiting faces are essentially in parallel with the lower face 105 of the upper plate portion 103. These blocks 117 are able to engage either underneath the lower edge of the infusion device or in suitable recesses on the side faces of the infusion device as such.

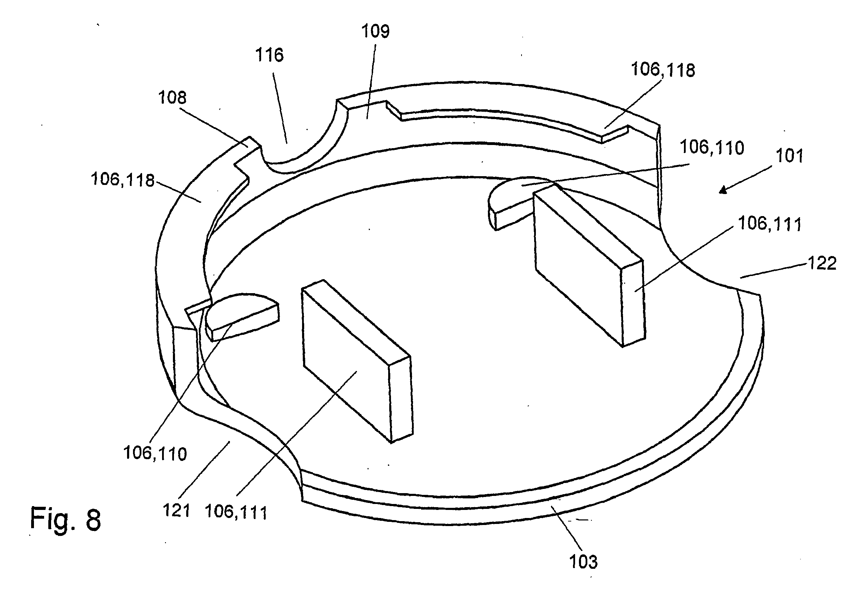

Figure 8 shows a third exemplary embodiment of a cover 101 according to the invention and wherein the difference between this and the teachings of Figures 5-6 is firstly that the attachment means 106 have plate elements 111 that do not in this case either comprise a concave delimiting edge 112. These attachment means 111 are thus true rectangular plates. Furthermore the flange 108 comprises essentially two wall portions 118 that extend perpendicular from the flange 108 and has the same material thickness as the flange as such. This edge, thus constituted by the wall portions 118, and which more or less corresponds to the delimiting edge of the flange, can like the taps or blocks 117 shown in Figure 7, engage either in convenient recesses on the infusion device or underneath this lower face. In this embodiment, preferably, the flange 108 is interrupted such that a circular recess 116 is provided. Furthermore, the geometrical shape of the upper plate element 103 is not completely circular; recesses 121, 122 having been provided that enable the fingers to more easily seize around the cover.

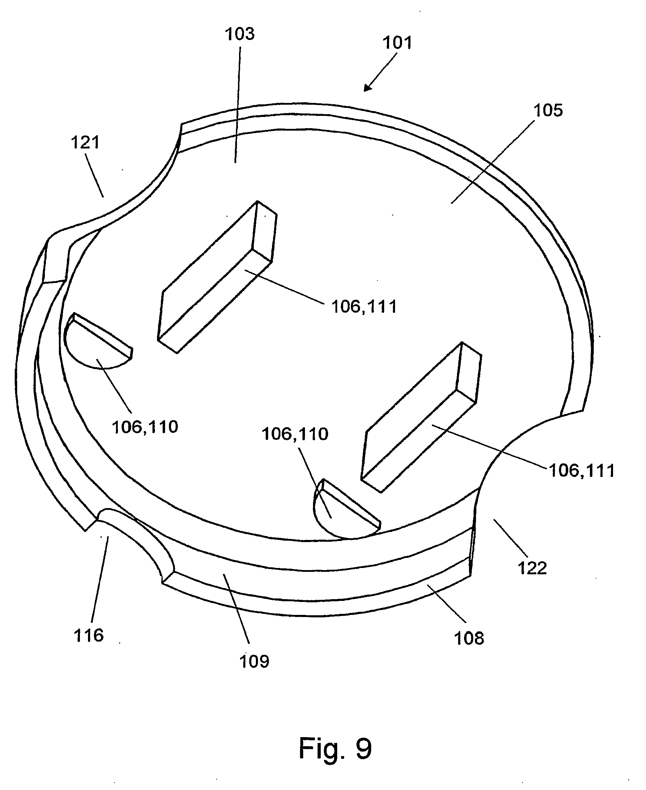

Figure 9 is essentially identical to the one shown in Figure 8, but wherein the perpendicular wall portion 118 extending from the flange 108 is not provided.

Conveniently the cover is made in an injection moulding process and wherein suitable materials are M-ABS and PC-ABS.

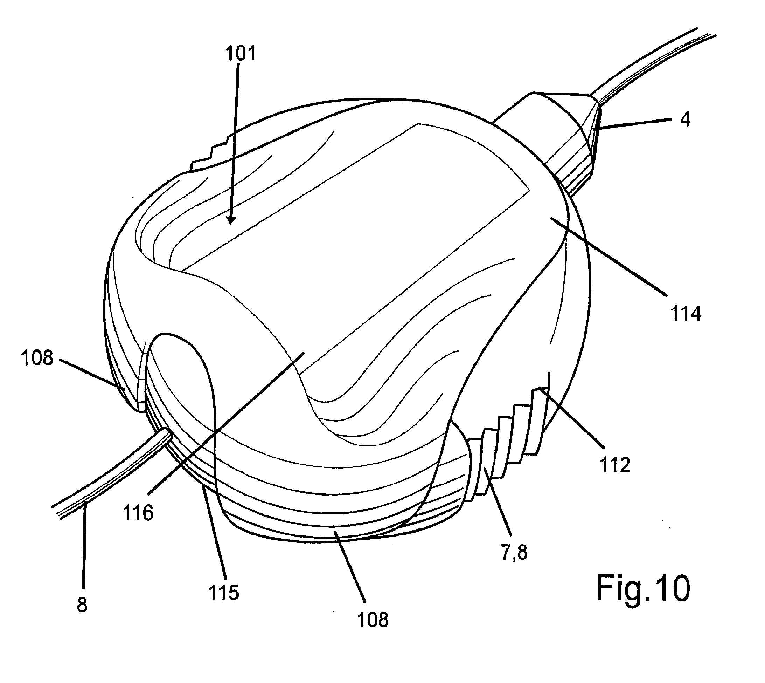

Figure 10 shows a cover 101 essentially like the one shown in Figures 5-6 located on an infusion device 102 corresponding to an infusion device 102 as taught in Figures 1-4. Thus the cover 101 is shown to cover any recesses and cut-outs present in the upper face of the infusion device, and wherein the flange 108 engages downwards around the rear side face of the infusion device 102 and corresponding to the rearmost face of the cannula housing 1 and with a circular recess 116 that ensures free access of the cannula 2 from the infusion device. When the infusion device is thus to be used, its recesses 7 and 8 are seized and the fingers are pressed towards each other on each their side. By this process the cannula housing 1 and the connection hub 3 are separated from each other. Due to the shape of the cover 101 and the engagement of the attachment means in relation to the infusion device 102, it remains in place on the cannula housing1 , while the connection hub 3 is separated off. Subsequently the infusion device 102 can be used as is also taught in eg US 5522803.

The cover of the present is not intended for use merely in connection with an infusion device as described above; of course, it may also lend itself to infusion devices having other geometries. The essential aspect is that the lower face of the cover has attachment means that engage with elements on the upper face of the infusion device, typically by a male/female engagement functionality, wherein the male part(s) are located on the lower face of the cover and wherein the female part(s) are the recesses and other cut-outs that are typically present on the upper face of an infusion device. Whether the upper face of the infusion device is curved or concave, flat or triangular is of lesser relevance, the cover being essentially manufactured with a plate element which is congruent with the face and thus covers the same completely or partially.

## Claims

1. A cover (101) suitable for being located on an infusion device, said infusion device being of the type comprising
- a cannula housing (1) having an upper face and a lower face, said lower face carrying an adhesive for securing said cannula housing (1) to the skin of a patient;
- at least one cannula (2) extending from said cannula housing (1); and
- a separate connector hub (3) being interconnectable with said cannula housing,
said cover (101) comprising an upper plate element (103) with an upper face (104) and a lower face (105) for orienting towards said upper face of the infusion device, **characterised in that** the lower face (105) of the plate element (103) comprises attachment means (106) for connecting said cover with said infusion device, said attachment means (106) cooperating with complementary elements of the infusion device, said cover (101) being sized to extend above said cannula housing (1) and said connector hub (3) when said attachment means (106) cooperate with said elements on the infusion device, and **in that** said cover (101) is disconnectable from said infusion device.

2. A cover according to claim 1, **characterised in that** the cover (101), when located on an infusion device of the type mentioned in claim 1, covers at least partially the connection between the cannula housing (1) and the connector hub (3).

3. A cover according to claim 1 or 2, **characterised in that** when said cover is located on an infusion device of the type mentioned in claim 1 the attachment means (106) cooperate with elements on the cannula housing (1).

4. A cover according to any of the claims 1-3, **characterised in that** the cover (101) further comprises a flange (108) that forms a first angle in relation to the upper plate (103) and is connected thereto.

5. A cover according to claim 4, **characterised in that** when said cover is located on an infusion device of the type mentioned in claim 1, the face of the flange (108) facing towards the outer side faces of the infusion device is essentially congruent therewith.

6. A cover according to any of the claims 3-4, **characterised in that** when said cover is located on an infusion device of the type mentioned in claim 1 the expanse of the upper plate element (103) is smaller than the expanse of the upper face of the infusion device, and that the flange (108) extends partially around the side faces of the infusion device.

7. A cover according to any one of the preceding claims, **characterised in that** the attachment means (106) are male parts and that the complementary elements on the infusion device are provided by recesses.

8. A cover according to any one of the preceding claims, **characterised in that** the attachment means (106) comprise at least two protruding partially cylindrical elements located (110) at a first mutual distance.

9. A cover according to any one of the preceding claims, **characterised in that** the attachment means (106) comprise at least two protruding plate elements (111) located at a second mutual distance.

10. A cover according to claim 7, **characterised in that** the protruding plate elements (111) are essentially rectangular plate-shaped and that when said cover is located on an infusion device of the type mentioned in claim 1, said protruding plate elements (111) are parallel to the centre axis of the infusion device.

11. A cover according to claims 7 or 8, **characterised in that** the protruding plate elements (111) comprise a concave delimiting edge (112) and concave side face (113).

12. A combination of an infusion device and a cover (101) according to any one of claims 1-11.

## Patentansprüche

1. Abdeckung (101), welche geeignet ist, an einer Infusionsvorrichtung angebracht zu werden, wobei die Infusionsvorrichtung von der Art ist, dass sie
- ein Gehäuse für eine Kanüle (1) mit einer oberen Seite und einer unteren Seite, wobei die untere Seite ein Klebemittel trägt, um das genannte Gehäuse für eine Kanüle an der Haut eines Patienten festzulegen;
- zumindest eine Kanüle (2), welche sich aus dem genannten Gehäuse für eine Kanüle (1) erstreckt; und
- einen separaten Anschlussteil (3), der mit dem genannten Gehäuse für eine Kanüle verbunden werden kann, umfasst,
wobei die Abdeckung (101) ein oberes Plattenelement (103) mit einer oberen Oberfläche (104) und einer unteren Oberfläche (105) umfasst, welche auf die genannte Oberseite der Infusionsvorrichtung gerichtet werden kann, **dadurch gekennzeichnet, dass** die untere Oberfläche (105) des Plattenelements (103) Befestigungsmittel (106) zur Befestigung der genannten Abdeckung an der genannten Infusionsvorrichtung umfasst, wobei die Befestigungsmittel (106) mit komplementären Bauteilen der Infusionsvorrichtung zusammenwirken, wobei die genannte Abdeckung (101) von einer solchen Größe ist, dass sie sich über das genannte Gehäuse für eine Kanüle (1) und den genannten Anschlussteil (3) erstreckt, wenn die genannten Befestigungsmittel (106) mit den genannten Elementen auf der Infusionsvorrichtung zusammenwirken und dass die genannte Abdeckung (101) von der genannten Infusionsvorrichtung abgenommen werden kann.

2. Abdeckung nach Anspruch 1, **dadurch gekennzeichnet, dass** die Abdeckung (101) zumindest teilweise die Verbindung zwischen dem Gehäuse für die Kanüle (1) und dem Verbindungsteil (3) bedeckt, wenn sie an der Infusionsvorrichtung des in Anspruch 1 genannten Typs befestigt ist.

3. Abdeckung nach Anspruch 1 oder 2, **dadurch gekennzeichnet, dass** die Befestigungsmittel (106) mit den Elementen auf dem Gehäuse für eine Kanüle (1) zusammenwirken, wenn die genannte Abdeckung an der Infusionsvorrichtung des in Anspruch 1 genannten Typs befestigt ist.

4. Abdeckung nach einem der Ansprüche 1 bis 3, **dadurch gekennzeichnet, dass** die Abdeckung (101) weiters einen Flansch (108) aufweist, welcher einen ersten Winkel bezüglich der oberen Platte (103) einschließt und mit dieser verbunden ist.

5. Abdeckung nach Anspruch 4, **dadurch gekennzeichnet, dass** die Oberfläche des Flansches (108), welche zu den äußeren Oberflächen der Infusionsvorrichtung weist, im Wesentlichen mit diesen kongruent ist, wenn die genannte Abdeckung an der Infusionsvorrichtung des in Anspruch 1 genannten Typs befestigt ist.

6. Abdeckung nach einem der Ansprüche 3 bis 4, **dadurch gekennzeichnet, dass** die Fläche des oberen Plattenelements (103) kleiner ist als die Fläche der oberen Oberfläche der Infusionsvorrichtung und dass sich der Flansch (108) teilweise um die Seitenflächen der Infusionsvorrichtung erstreckt, wenn die genannte Abdeckung an der Infusionsvorrichtung des in Anspruch 1 genannten Typs befestigt ist.

7. Abdeckung nach einem der vorhergehenden Ansprüche, **dadurch gekennzeichnet, dass** die Befestigungsmittel (106), männliche Teile sind und dass die komplementären Elemente an der Infusionsvorrichtung von Ausnehmungen gebildet sind.

8. Abdeckung nach einem der vorhergehenden Ansprüche, **dadurch gekennzeichnet, dass** die Befestigungsmittel (106) zumindest zwei vorstehende, teilweise zylindrische Elemente (110) aufweisen, welche in einer ersten Distanz voneinander angeordnet sind.

9. Abdeckung nach einem der vorhergehenden Ansprüche, **dadurch gekennzeichnet, dass** die Befestigungsmittel (106), zumindest zwei vorstehende Plattenelemente (111) umfassen, welche in einer zweiten Distanz voneinander angeordnet sind.

10. Abdeckung nach Anspruch 7, **dadurch gekennzeichnet, dass** die vorstehenden Plattenelemente (111) im Wesentlichen rechteckig plattenförmig sind und dass die genannten vorstehenden Plattenelemente (111) parallel zur zentralen Achse der Infusionsvorrichtung sind, wenn die genannte Abdeckung an der Infusionsvorrichtung des in Anspruch 1 genannten Typs befestigt ist.

11. Abdeckung nach Anspruch 7 oder 8, **dadurch gekennzeichnet, dass** die vorstehenden Plattenelemente (111) eine konkave Begrenzungskante (112) haben und eine konkave seitliche Oberfläche (113) umfassen.

12. Kombination einer Infusionsvorrichtung und einer Abdeckung (101) nach einen der Ansprüche 1 bis 11.

## Revendications

1. Couvercle (101) convenant pour le placement sur un dispositif de perfusion, ledit dispositif de perfusion étant du type comprenant
- un boîtier pour canule (1) ayant une face supérieure et une face inférieure, ladite face inférieure portant un adhésif pour fixer ledit boîtier de canule (1) à la peau d'un patient;
- au moins une canule (2) s'étendant dudit boîtier de canule (1); et
- un concentrateur de connexion séparé (3) interconnectable audit boîtier de canule,
ledit couvercle (101) comprenant un élément plaque supérieur (103) avec une face supérieure (104) et une face inférieure (105) à orienter vers ladite face supérieure du dispositif à perfusion, **caractérisé en ce que** la face inférieure (105) de l'élément plaque (103) comprend des moyens de fixation (106) pour connecter ledit couvercle audit dispositif à perfusion, lesdits moyens de fixation (106) coopérant avec des éléments complémentaires du dispositif à perfusion, ledit couvercle (101) étant dimensionné pour s'étendre au-dessus dudit boîtier de canule (1) et dudit concentrateur de connexion (3) lorsque lesdits moyens de fixation (106) coopèrent avec lesdits éléments sur le dispositif à perfusion, et **en ce que** ledit couvercle (101) est déconnectable dudit dispositif à perfusion.

2. Couvercle suivant la revendication 1, **caractérisé en ce que** le couvercle (101), lorsqu'il est situé sur un dispositif à perfusion du type mentionné dans la revendication 1, recouvre au moins partiellement la connexion entre le boîtier de canule (1) et le concentrateur de connexion (3).

3. Couvercle suivant la revendication 1 ou 2, **caractérisé en ce que**, lorsque ledit couvercle est situé sur un dispositif à perfusion du type mentionné dans la revendication 1, les moyens de fixation (106) coopèrent avec des éléments sur le boîtier de canule (1).

4. Couvercle suivant l'une quelconque des revendications 1-3, **caractérisé en ce que** le couvercle (101) comprend en outre une bride (108) qui forme un premier angle en relation avec la plaque supérieure (103) et est connecté à celle-ci.

5. Couvercle suivant la revendication 4, **caractérisé en ce que**, lorsque ledit couvercle est situé sur un dispositif à perfusion du type mentionné dans la revendication 1, la face de la bride (108) faisant face aux faces latérales extérieures du dispositif à perfusion est essentiellement congruente à celles-ci.

6. Couvercle suivant l'une quelconque des revendications 3-4, **caractérisé en ce que**, lorsque ledit couvercle est situé sur un dispositif à perfusion du type mentionné dans la revendication 1, la dilatation de l'élément plaque supérieur (103) est plus faible que la dilatation de la face supérieure du dispositif à perfusion et **en ce que** la bride (108) s'étend partiellement autour des faces latérales du dispositif à perfusion.

7. Couvercle suivant l'une quelconque des revendications précédentes, **caractérisé en ce que** les moyens de fixation (106) sont des parties mâles et **en ce que** les éléments complémentaires sur le dispositif à perfusion sont fournis par des renfoncements.

8. Couvercle suivant l'une quelconque des revendications précédentes, **caractérisé en ce que** les moyens de fixation (106) comprennent au moins deux éléments partiellement cylindriques en saillie (110) écartés mutuellement d'une première distance.

9. Couvercle suivant l'une quelconque des revendications précédentes, **caractérisé en ce que** les moyens de fixation (106) comprennent au moins deux éléments plaques en saillie (111) écartés mutuellement d'une deuxième distance.

10. Couvercle suivant la revendication 7, **caractérisé en ce que** les éléments plaques en saillie (111) sont essentiellement en forme de plaque rectangulaire et **en ce que**, lorsque ledit couvercle est situé sur un dispositif à perfusion du type mentionné dans la revendication 1, lesdits éléments plaques en saillie (111) sont parallèles à l'axe central du dispositif à perfusion.

11. Couvercle suivant la revendication 7 ou 8, **caractérisé en ce que** les éléments plaques en saillie (111) comprennent un bord concave de délimitation (112) et une face latérale concave (113).

12. Combinaison d'un dispositif à perfusion et d'un couvercle (101) suivant l'une quelconque des revendications 1-11.
